# EUROPEAN PATENT APPLICATION

(11) **EP 4 481 384 A1**
(43) Date of publication of application: **25.12.2024**
(21) Application number: 23756474.5
(22) Date of filing: 17.02.2023
(51) Int. Cl.: G01N 33/531, C12N 15/11, C12Q 1/37, C12Q 1/42

(54) **NONSPECIFIC REACTION INHIBITOR**

(30) Priority: 18.02.2022 JP 2022023357
(71) Applicant: PHC Corporation, Toon-shi Ehime 791-0395 (JP)
(72) Inventor: YAMAGUCHI KOJIMA Kanako, Toon-shi, Ehime 791-0395 (JP); YOSHIDA Tatsuya, Toon-shi, Ehime 791-0395 (JP)
(74) Representative: Gille Hrabal Partnerschaftsgesellschaft mbB Patentanwälte
(86) International application number: PCT/JP2023/005758
(87) International publication number: WO 2023/157950

(57) **Abstract**

Provided is a non-specific reaction inhibitor that has high solubility, can be added in sufficient amounts, is inexpensive, and has small variation among different lots. The non-specific reaction inhibitor of the present invention comprises a complex of a peptide or nucleic acid molecule that specifically binds to a non-specific reaction substance and a polymer compound.

## Description

### TECHNICAL FIELD

The present invention relates to a non-specific reaction inhibitor for suppressing non-specific reactions that are an obstacle to accurate detection and quantification of a trace substance in immunological measurement.

### BACKGROUND ART

JP 11-287801 A discloses a method for inhibiting non-specific reactions by making an antibody against non-specific reactive substances coexist in a measurement reagent, thereby reducing non-specific reactions caused by non-specific reactive substances in samples. However, this technique has a problem that when IgG or IgM is used as an antibody against non-specific reactive substances, immune complexes are formed with the non-specific reactive substances, resulting in an immunonephelometric reaction, and accurate measurement values may not be obtained when using latex immunoturbidimetry. To address this issue, the amount of IgG or IgM added to the nonspecific reaction substances was reduced, but this sometimes resulted in an insufficient non-specific inhibitory effect.

In order to eliminate the immunonephelometric reaction, IgG is fragmented into F(ab')₂ to reduce its hydrophobicity, which allows a large amount to be added to the reagent and makes it difficult for the immunonephelometric reaction to occur. However, there was an issue with the durability of the non-specific inhibitory effect in the reagent, which was thought to be due to the weakening of the non-specific inhibitory effect caused by the decomposition of F(ab')₂ to Fab'. JP 5189098 B indicates that the low non-specific inhibitory effect of Fab' may be due to an insufficient molecular size, and to solve this problem, a method is disclosed in which antibody fragments such as Fab' are modified with a polymer compound to enlarge them, thereby enhancing the non-specific inhibitory effect.

### CITATION LIST

### PATENT LITERATURE

[Patent literature 1] JP H11-287801 A
[Patent literature 2] JP 5189098 B

### SUMMARY OF INVENTION

### TECHNICAL PROBLEM

However, in recent years, there has been a demand for more sensitive immunoassays, and as the amount of sample used increases in an effort to increase the sensitivity of reagents, the amount of non-specific reaction substances present in the reaction system increases significantly, making it necessary to include a large amount of non-specific reaction inhibitor in the reagents. Similarly, when a large amount of non-specific substances are present in the sample, it is necessary to add a large amount of non-specific reaction inhibitor to the reagent. Under these circumstances, the technology of JP 5189098 B uses relatively expensive biological raw materials, such as antibodies, antibody fragments, and the like, which poses problems in terms of cost and lot-to-lot differences in raw materials. In addition, because the essence of the technology is the enlargement of molecular size, the effect of improving solubility in the reagent is insufficient, and when the sample amount is increased in an effort to increase sensitivity, it is not satisfactory in terms of ensuring the amount of addition sufficient to obtain a sufficient non-specific inhibitory effect.

In view of these circumstances, an object of the present invention is to provide a non-specific reaction inhibitor that is highly soluble, can be added in sufficient amounts, is inexpensive, and has little lot-to-lot variation.

### SOLUTION TO PROBLEM

As a result of extensive research into achieving the above object, it was discovered that by modifying a peptide that is even smaller in molecular weight than Fab' and has the ability to bind to non-specific reaction substances with a polymer such as polyethylene glycol (PEG) or the like, an effective non-specific inhibitory effect could be achieved. Fab' is a molecule with a molecular weight of about 55 kDa, but according to the description in JP 5189098 B, even with a molecular weight of that size, the non-specific inhibitory effect is lost. However, the inventors unexpectedly discovered that even a very small peptide of about 1 kDa can have an effective non-specific inhibitory effect by binding about 5 kDa PEG, even though the molecular weight is smaller than that of Fab'.

The above object of the present invention can be achieved by the following inventions:
[1] A non-specific reaction inhibitor for immunological measurement, comprising a complex of a peptide or nucleic acid molecule that specifically binds to a non-specific reaction substance and a polymer compound.
[2] The non-specific reaction inhibitor of [1], wherein the substance that specifically binds to the non-specific reaction substance is a chemically synthesizable substance.
[3] The non-specific reaction inhibitor of [1] or [2], wherein the complex is 50 kDa or less.
[4] The non-specific reaction inhibitor of any one of [1] to [3], wherein the polymer compound is polyethylene glycol.
[5] The non-specific reaction inhibitor of any one of [1] to [4], wherein the non-specific reaction substance is an immunoglobulin.
[6] An immunological measurement method, characterized by using a complex of a peptide or nucleic acid molecule that specifically binds to a non-specific reaction substance and a polymer compound.
[7] The immunological measurement method of [6], wherein the immunological measurement method is a latex agglutination photometric assay, an immunonephelometric assay, an immunoturbidimetric assay, a chemiluminescent immunoassay, an enzyme immunoassay, a fluorescent immunoassay, or a radioimmunoassay.
[8] An immunological measurement reagent, comprising a complex of a peptide or nucleic acid molecule that specifically binds to a non-specific reaction substance and a polymer compound.
[9] The immunological measurement reagent of [8], wherein the complex of a peptide or nucleic acid molecule that specifically binds to a non-specific reaction substance and a polymer compound is contained in a stabilizing reagent.

### ADVANTAGEOUS EFFECTS OF INVENTION

According to the present invention, the cost is lower than that of biological raw materials, and lot-to-lot differences can be reduced because chemical synthesis is possible. Furthermore, according to the present invention, because the molecular weight is lower, the solubility is high and it is possible to ensure a sufficient amount of addition.

### BRIEF DESCRIPTION OF THE DRAWINGS

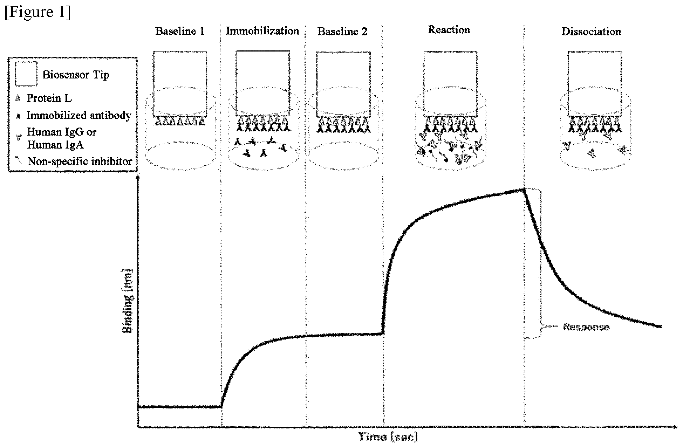

[Fig. 1] Figure 1 shows each step of the measurement using Octet (trademark) R2.

### DESCRIPTION OF EMBODIMENTS

The non-specific reaction inhibitor of the present invention comprises a complex of a peptide or nucleic acid molecule that specifically binds to a non-specific reaction substance (hereinafter sometimes referred to as a binding partner) and a polymer compound.

The term "non-specific reaction substance" as used herein is a substance that induces non-specific reactions in immunological measurements that utilize an antigen-antibody reaction. Specific factors when human body fluids are used as samples include human IgM, human IgG, human IgA, human IgE, human IgD, and factors that bind to the antibodies, such as complement, rheumatoid factor, Fc receptor, and the like, and when body fluids from an animal other than humans as samples, include IgM, IgG, IgA, IgE of the animal, and factors that bind to the antibodies, and the like.

The immunological measurements include a latex agglutination photometric assay, an immunonephelometric assay, an immunoturbidimetric assay, a chemiluminescent immunoassay, an enzyme immunoassay, a fluorescent immunoassay, a radioimmunoassay, and the like. Measurements that do not have a B/F separation step, such as a latex agglutination photometric assay, an immunonephelometric assay, an immunoturbidimetric assay, and the like, are particularly susceptible to the effects of non-specific reaction substances. In such measurements, the concentration of a test sample during the antigen-antibody reaction is often a few percent, and the higher the concentration of the test sample, the greater the amount of non-specific reaction inhibitor that must be added. Even if the concentration of the test sample in the reaction solution is higher than conventional concentrations (4% or more, 5% or more, or 8% or more), the non-specific reaction inhibitor of the present invention can be added in a sufficient amount. All of these utilize antigen-antibody reactions, and there are two types of antibodies that detect target antigens: polyclonal antibodies and monoclonal antibodies. In any of the measurements, the non-specific reaction inhibitor of the present invention may be added to the test sample before or during the antigen-antibody reaction, preferably before the antigen-antibody reaction is performed.

The binding partner used in the present invention that specifically binds to a non-specific reaction substance may be a peptide or a nucleic acid molecule (for example, an aptamer), but the present invention is characterized in that an antibody, or an antibody fragment having a molecular weight of higher than that of Fab' is not used. The molecular weight of the binding partner is typically 0.5 to 47 kDa, preferably 0.8 to 15 kDa, and more preferably 1 to 8 kDa. In the present invention, by using a binding partner with a molecular weight smaller than that of Fab', it is possible to ensure high solubility and sufficient addition amount. According to the present invention, it is possible to increase the amount of non-specific reaction inhibitor added to the reaction system in which an immune complex is formed, and as a result, it is possible to handle test samples with high concentrations of non-specific reaction substances, and it is also possible to increase the concentration of the test sample in the reaction system.

The binding partner used in the present invention is preferably a substance that can be chemically synthesized. Since the present invention does not use an antibody or an antibody fragment, it is possible to provide a non-specific reaction inhibitor that is inexpensive and has little lot-to-lot variation. The binding partner may contain a functional group for modifying a polymer compound, biotin, a spacer, and the like as appropriate.

Examples of the non-specific reaction inhibitor of the present invention (i.e., a complex between a binding partner of a non-specific reaction substance and a polymer compound) include a chemically modified peptide or nucleic acid molecule chemically modified with a polymer compound, or a complex between a peptide or nucleic acid molecule and a polymer compound bound by a biotin-avidin bond.

In the above chemical modification, for example, thiol groups, amino groups, hydroxyl groups, or carboxyl groups can be targeted and linked via "reactive derivatives".

The "reactive derivatives" used in the modification targeting thiol groups include, for example, thiol-selective reactive groups such as maleimides and vinyl sulfones. In addition, reactive derivatives may be directly bonded to the polymer, or crosslinkers containing reactive derivatives may be used.

The "Reactive derivatives" used in the modification targeting amino groups include, for example, N-hydroxysuccinimide (NHS) esters, N-hydroxysulfosuccinimide (Sulfo-NHS) esters, and the like. In addition, there are compounds containing aldehyde groups (for example, glutaraldehyde), polymers that already contain aldehyde groups, and the like.

Modifications targeting carboxyl groups include, for example, complexes obtained by reacting amino groups with carbodiimide (1-Ethyl-3-[3-dimethylaminopropyl]carbodiimidehydrochloride) as a catalyst.

Modifications targeting hydroxyl groups can be prepared, for example, using compounds containing isocyanic acid derivatives.

Polymers in which reactive derivatives are incorporated may be obtained commercially (for example, NOF Corporation) or may be prepared using conventional chemical methods.

The present invention also includes a method that utilizes a binding mode that has high affinity but is not a covalent bond between the binding partner and the polymer, such as biotin and avidin.

Polymer compounds that can be used in the present invention include, for example, polysaccharides, proteins, and organic polymers.

The polysaccharides include, for example, dextran, dextrin, agarose, carboxymethyl (CM)-cellulose, heparin, soluble starch, and the like. The polysaccharides may be either linear or branched. To modify the binding partner using polysaccharides, the conventional methods such as a periodate oxidation method, a cyanogen bromide method, a carbodiimide method, a cyanuric chloride method, an epichlorohydrin method, an SPDP (N-Succinimidyle 3-[2-pyridyldithio]propionate) reagent method, an active ester method, or the like can be used. Polysaccharides in which reactive derivatives are incorporated can be obtained commercially or prepared using conventional chemical methods.

The above proteins may be a complex in which multiple amino acids are linked by peptide bonds, for example, one purified from animals, one artificially prepared by genetic engineering, or one prepared by chemical synthesis, such as a synthetic peptide. The proteins include, for example, casein, milk casein, gelatin, recombinant albumin, and the like. Polyamino acids include homopolymers of arginine, lysine, glutamic acid, and the like, and random polymers of lysine and glycine, lysine and serine, and the like. Examples of a method for binding proteins include binding a crosslinker to the amino, carboxyl, sulfide groups, or the like of the protein as targets, and then binding the protein to a binding partner via the crosslinker. Another method includes using a carbodiimide as a catalyst to bind the protein to a binding partner. In the present invention, a preferred method includes reacting the amino groups of a protein with a crosslinking agent such as EMCS [N-(6-Maleimidocaproyloxy)succinimide; Dojin] or SMCC [succinimdyl 4-(N-maleimidomethyl)cyclohexane carbonate; Dojin], and then binding the crosslinking agent to a binding partner having a sulfide group at its end. The protein with a reactive derivative introduced therein, which is used to prepare the product of the present invention, may be obtained commercially or may be prepared using conventional chemical methods.

Examples of the organic polymers include polyethylene glycol, polyvinyl alcohol, polyallyl alcohol, polyethyleneimine, polymethyl methacrylate, polyacrylic acid, polyallylamine, and polysaccharides. The organic polymer may be a straight chain or a branched chain, or may be a random copolymer of multiple types. It may also be a synthetic polymer with a spherical structure such as a dendrimer. The polymer may be synthetic or naturally derived.

Polyethylene glycol is a polymeric compound based on the structure of polymerized ethylene glycol. Other functional groups can be introduced into the hydroxyl groups of polyethylene glycol, and these functional groups can be used to bind to antibodies. Activation methods for binding polyethylene glycol to antibodies include, for example, methods using cyanuric chloride, carbodiimidazole, N-hydroxysuccinimide, carbodiimide, and the like. As the polyethylene glycol having functional groups introduced therein, commercially available products can be used. By using commercially available polyethylene glycol having maleimide groups, succinimide groups, amino groups, sulfide groups, or the like introduced therein, the preparation can be carried out efficiently. Polyethylene glycols with introduced maleimide or succinimide groups are more preferred because they have good binding efficiency with binding partners having sulfide or amino groups at the terminus. The polyethylene glycol may be linear or branched, and may include polyethylene glycols in which a part of the polyethylene glycol is substituted with other chemical structures or polyethylene glycols modified with other polymers or compounds.

As a method for chemical modification with organic polymers other than polyethylene glycol, there is a method in which functional groups are introduced into the organic polymer and then bonded to the antibody, similar to the above-mentioned method for chemical modification with polyethylene glycol. In the case of organic polymers that already contain reactive derivatives, it may not be necessary to introduce new functional groups into the organic polymers during the preparation stage. In the present invention, it is preferable to use polymers into which maleimide groups, succinimide groups, amino groups, or carboxyl groups have been introduced. Organic polymers into which reactive derivatives have been introduced may be obtained commercially or may be prepared using conventional chemical methods.

The molecular size of the polymer compound is not particularly limited, but generally has an average molecular weight of about 200 Da to 1000 kDa, for example, 1 kDa to 1000 kDa, preferably 10 kDa to 100 kDa. In the case of polyethylene glycol, the molecular size is preferably 3 kDa to 49.5 kDa. Depending on the type of polymer used, the molecular size can be appropriately selected taking into consideration hydrophilicity, three-dimensional structure, non-specific inhibitory effect, and the like.

The non-specific reaction inhibitor of the present invention can be used by adding a complex of a binding partner, which is the active ingredient, and a polymer compound to an immunoassay system. Specifically, a solution of a binding partner modified with a polymer compound is prepared, and before reacting an antibody against the antigen to be measured with the antigen, the above solution is added to the sample in advance to react the non-specific reaction substance with its corresponding binding partner, thereby suppressing the non-specific reaction caused by the non-specific reaction substance; alternatively, a binding partner modified with a polymer compound is included in a solution of an antibody against the antigen to be measured, and this solution is added to the sample to react the non-specific reaction substance with its corresponding binding partner, thereby suppressing the non-specific reaction caused by the non-specific reaction substance.

The immunological measurement reagent of the present invention may be any reagent that further contains the non-specific reaction inhibitor of the present invention in addition to a conventional immunological measurement reagent that utilizes an antigen-antibody reaction, and is composed of one liquid or two or more liquids.

When it is composed of one liquid, it comprises at least a reaction reagent containing a support carrying an antigen or antibody for forming an immunological complex. According to a known method, a sample is reacted with the reagent and a signal is detected. The non-specific reaction inhibitor of the present invention may be added to the reagent before the sample reacts with the reagent, but is preferably supplied in a state where it is added to the reagent. When it is composed of two or more liquids, it consists of a stabilizing reagent and a reaction reagent containing a support carrying at least an antibody or antigen for forming an immunological complex. The stabilizing reagent is a reagent for diluting the sample to an appropriate concentration or for pretreating the sample, and can be prepared according to a known method. According to a known method, the sample is reacted with the stabilizing reagent, and then with the reaction reagent, and the signal is detected. The non-specific reaction inhibitor of the present invention may be added to the stabilizing reagent and/or the reaction reagent before the sample reacts with the reaction reagent, and is preferably supplied in a state where it has been added to the stabilizing reagent and/or the reaction reagent. It is particularly preferable that the non-specific reaction inhibitor is added to the stabilizing agent.

Immunological measurement reagents are used to measure, for example, elastase, cystatin C, sEs (soluble E-selectin), SF (soluble fibrin), PC (protein C), PPI (plasmin plasmin inhibitor), cTn (thrombomodulin), myoglobin, CK-MB, BNP (B-type natriuretic peptide), NT-proBNP (N-terminal pro-BNP), cTnI (cardiac troponin I), AFP (alpha-fetoprotein), β2m (β-2-microglobulin), CEA (carcinoembryonic antigen), ferritin, CA19-9 (carbohydrate antigen 19-9), PAP (prostatic acid phosphatase), PSA (prostate-specific antigen), CRP (C-reactive protein), Mb (myoglobin), PCT (procalcitonin), presepsin, IL-2R (interleukin 2 receptor), RF (rheumatoid factor), ASO (antistreptolysin-O), FDP (fibrin degradation products), ATIII (antithrombin III), TAT (thrombin-antithrombin III complex), plasminogen, α2PI (α-2-plasmin inhibitor), D-dimer (fibrin degradation product D-fragment dimer), IgG (immunoglobulin G), IgA (immunoglobulin A), IgM (immunoglobulin M), IgE (immunoglobulin E), C3 (complement component 3), C4 (complement component 4), urinary albumin, hCG (human chorionic gonadotropic hormone), hPL (human placental lactogen), insulin, HBs antigen (hepatitis B surface antigen), HBs antibody (anti-hepatitis B surface antigen antibody), HBc antibody (anti-hepatitis B core antigen antibody), HCV antibody (anti-hepatitis C virus antibody), Treponema (anti-Treponema pallidum antibody), TSH (thyroid stimulating hormone), LH (luteinizing hormone), FSH (follicle stimulating hormone), prolactin, testosterone, estradiol, progesterone, digoxin, digitoxin, quinidine, procainamide, NAPA (N-acetylprocainamide), theophylline, phenytoin, phenobarbital, carbamazepine, valproic acid, ethosuccinimide, gentamicin, tobramycin, amikacin, zinkomicin, cyclosporine A, B12 (vitamin B12), folic acid, T3 (triiodothyronine), T4 (thyroxine), and estrogen.

### EXAMPLES

Chemically synthesized peptides or nucleic acid aptamers were used as small molecules that bind to non-specific reaction substances, and polyethylene glycol (hereinafter referred to as PEG) was used as a polymer compound to modify them. The present invention will now be further illustrated by, but is by no means limited to, the following Examples.

### «Example 1: Preparation of PEG-modified peptides»

One molecule of PEG was attached to one molecule of peptide via the amino group at the N-terminus. The peptides used were a human IgG-binding peptide of approximately 1.5 kDa (amino acid sequence: DCAWHLGELVWCT: SEQ ID NO: 1), which is known for the purification of human IgG, and a human IgA-binding peptide of approximately 1.8 kDa (amino acid sequence: HMVCLSYRGRPVCFSL: SEQ ID NO: 2), which is reported for the purification of human IgA. PEG with a succinimide group at one end (molecular weight 20 kDa; manufactured by NOF CORPORATION) was used.

### [Preparation of PEG-modified IgG-binding peptide]

After dissolving the human IgG-binding peptide in 0.2 mol/L NaHCO₃ (pH 8.5) to a concentration of 1.0 mg/mL, PEG with a succinimide group at one end was added to the peptide in equal molar amounts, and the reaction was carried out overnight at 4°C while stirring on a rotator. After the reaction, the mixture was passed through a human IgG affinity column using phosphate buffered saline (PBS) as the running buffer to remove unreacted PEG. The PEG-modified peptide was eluted with 0.2 mol/L citrate buffer (pH 3.0). Concentration of the PEG-modified peptide and removal of unreacted peptide were carried out by ultrafiltration, and the buffer was replaced with PBS. Concentration was measured using a Quantitative Colorimetric Peptide Assay (manufactured by Thermo Fisher), and the PEG-modified peptide was stored at 4°C.

### [Preparation of PEG-modified IgA-binding peptide]

After dissolving the human IgA-binding peptide in PBS (pH 7.4) containing 40% dimethyl sulfoxide (DMSO) to a concentration of 1.0 mg/mL, PEG with a succinimide group at one end was added to the peptide in equal molar amounts, and the reaction was carried out overnight at 4°C while stirring on a rotator. After the reaction, the mixture was passed through a human IgA affinity column using PBS as the running buffer to remove unreacted PEG. The PEG-modified peptide was eluted with 0.2 mol/L citrate buffer (pH 3.0). Concentration of the PEG-modified peptide and removal of unreacted peptide were carried out by ultrafiltration, and the buffer was replaced with PBS. Concentration was measured using a Quantitative Colorimetric Peptide Assay (manufactured by Thermo Fisher), and the PEG-modified peptide was stored at 4°C.

### «Example 2: Confirmation of reaction inhibitory effect on human IgG or human IgA with or without PEG modification»

As a model system to confirm the non-specific inhibitory effect, an anti-human IgG antibody and human IgG, or an anti-human IgA antibody and human IgA were used, and the reaction inhibitory effect on the IgG-binding peptide or the IgA-binding peptide with smaller molecular weights than antibody fragments, with or without PEG modification, was confirmed.

### [Methods]

The measurements were performed using Bio-Layer Interferometry (BLI) using Octet(trademark) R2 (manufactured by SARTORIUS), which was a biomolecular interaction analysis system. The basic principles of the Bio-Layer Interferometry are briefly explained below. When light of a specific wavelength is projected onto a layer of biomolecules fixed on the surface of a sensor chip, the light is reflected from both the surface of the biomolecular layer and the internal reference layer, resulting in an optical interference wave. When molecules in the measurement sample bind to the biomolecules on the surface of the sensor chip, the thickness of the layer at the tip of the sensor increases, causing a wavelength shift in the interference wave. By measuring this change in wavelength shift, it is possible to quantify and kinetically analyze in real time the number of molecules that bind to the biomolecules immobilized on the sensor chip surface. In this Example, the test sample and a non-specific reaction inhibitor were allowed to coexist in the measurement sample, and human IgG or human IgA was absorbed in the sample solution. The measurements were carried out according to the operating manual attached to the OctetR2.

### [Confirmation of reaction inhibitory effect on human IgG]

As an antibody solution, an anti-human IgG polyclonal rabbit antibody (manufactured by Dako) was diluted with PBS(-) to 25 µg/mL to prepare an anti-human IgG antibody solution. As a sample solution, the test sample was human IgG adjusted to 1 mg/mL in PBS(-), and to 20 µL of this test sample, the IgG-binding peptide or the IgG-binding peptide modified with 20 kDa PEG was added to a final concentration of 25 µg/mL or 50 µg/mL, respectively, and the total volume was adjusted to 200 µL to prepare the first reagent (hereinafter referred to as R1) of LPIA Genesis TAT (manufactured by LSI Medience Corporation), which was a thrombin-antithrombin III complex (hereinafter referred to as TAT) measurement reagent.

The sample solution and the anti-human IgG antibody solution were added to the designated wells of a 96-well black plate (manufactured by Greiner Bio-One) at 200 µL per well. To the baseline, dissociation and washing wells, 200 µL of PBS(-) was added. This plate and a biosensor (Biosensor/ProL: manufactured by SARTRIUS) were placed in the designated positions of the OctetR2. After operating the OctetR2 under the conditions shown in Table 1 below and acquiring data, the wavelength shift change (Response) was calculated using the analysis software included with the OctetR2. The measurements were carried out at a temperature of 30°C.

**[Table 1]**

| | Step | Contact time [sec] | Speed [rpm] |
|---|---|---|---|
| 1 | Baseline 1 | 60 | 1000 |
| 2 | Immobilization of antibody | 60 | 1000 |
| 3 | Baseline 2 | 60 | 1000 |
| 4 | Reaction with sample solution | 120 | 1000 |
| 5 | dissociation | 180 | 1000 |
| Steps 1 to 5 are repeated until all samples have been measured. | | | |

### [Confirmation of reaction inhibitory effect on human IgA]

As an antibody solution, an anti-human IgA polyclonal rabbit antibody (manufactured by Dako) was diluted with PBS(-) to 25 µg/mL to prepare an anti-human IgA antibody solution. As a sample solution, the test sample was human IgA adjusted to 250 µg/mL in PBS(-), and to 20 µL of this test sample, the IgA-binding peptide or the IgA-binding peptide modified with 20 kDa PEG was added to a final concentration of 25 µg/mL or 50 µg/mL, respectively, and the total volume was adjusted to 200 µL to prepare the R1.

Measurements using OctetR2 were performed in the same manner as for confirming the reaction inhibitory effect on human IgG.

### [Results]

The reaction inhibitory effect on human IgG is shown in Table 2, and the reaction inhibitory effect on human IgA is shown in Table 3. The IgG-binding peptide or IgA-binding peptide modified with PEG had a stronger reaction inhibitory effect on the reaction between anti-human IgG antibody and human IgG, or between anti-human IgA antibody and human IgA, than the unmodified peptide. In addition, the IgG-binding peptide or IgA-binding peptide was 1-2 kDa, and even when modified with 20 kDa PEG, the molecular weight was 21-22 kDa, which was smaller than that of the antibody fragments, but it was found that they showed a reaction inhibitory effect.

The reason why the measured value increased by the addition of peptides not modified with PEG is that although the peptides bound to human IgG or human IgA, no steric hindrance was generated that was strong enough to prevent the binding of the anti-human IgG antibody to human IgG or the anti-human IgA antibody to human IgA, and the human IgG bound to the IgG-binding peptide bound to the anti-human IgG antibodies, and the human IgA bound to the IgA-binding peptide bound to the anti-human IgA antibodies, resulting in an increase in the thickness of the layer at the tip of the sensor and a larger change in wavelength shift than when only human IgG or human IgA bound to anti-human IgG or anti-human IgA antibodies when no non-specific reaction inhibitor was added.

**[Table 2]**

| Human IgG | | | |
|---|---|---|---|
| | Amount added | IgG-binding peptide | PEG-modified IgG-binding peptide |
| Response [nm] | 0 µg/mL | 6.6462 | 6.6462 |
| | 25 µg/mL | 7.4221 | 3.1484 |
| | 50 µg/mL | 7.6293 | 2.6181 |

**Table 3**

| Human IgA | | | |
|---|---|---|---|
| | Amount added | IgA-binding peptide | PEG-modified IgA-binding peptide |
| Response [nm] | 0µg/mL | 2.1381 | 2.1381 |
| | 25µg/mL | 2.1805 | 1.6061 |
| | 50µg/mL | 2.2263 | 1.5374 |

### «Example 3: Confirmation of non-specific reaction inhibitory effect with or without PEG modification»

Using human plasma, which exhibits non-specific reactions, we investigated the non-specific reaction inhibitory effect of an IgG-binding peptide, which have molecular weights smaller than those of antibody fragments, with and without PEG modification.

### [Methods]

As a test sample, sample A, which was human plasma that exhibited a non-specific reaction in the LPIA Genesis TAT (manufactured by LSI Medience Corporation), and characterized in that its non-specific reaction substance was IgG, was used.

The measurement was carried out under the same assay conditions as described in Example 2. In this Example, sample A and a non-specific reaction inhibitor were allowed to coexist in the measurement sample, and the non-specific reaction substances were absorbed in the sample solution.

The antibody solution used was an anti-TAT antibody solution prepared by diluting the anti-TAT antibody used in the second reagent (hereinafter referred to as R2) of LPIA Genesis TAT, which was characterized by containing latex particles to which a monoclonal antibody with specific binding ability to TAT was immobilized, with PBS(-) to a concentration of 25 µg/mL. The sample solution was prepared by adding a final concentration of 25 µg/mL or 50 µg/mL of IgG-binding peptide or 20kDa PEG-modified IgG-binding peptide as a non-specific reaction inhibitor to 20 µL of the test sample diluted 10-fold with PBS(-), and adjusting the total volume to 200 µL with R1.

### [Results]

The results are shown in Table 4. As in the results of Example 2, the IgG-binding peptide modified with PEG had a greater effect in suppressing the non-specific reaction than the unmodified peptide. Furthermore, although the IgG-binding peptide modified with PEG had a smaller molecular weight than those of antibody fragments, it showed the non-specific reaction inhibitory effect.

The reason why the measured value increased by the addition of the peptide not modified with PEG is that although the IgG-binding peptide bound to the non-specific reaction substances, no steric hindrance was generated that was strong enough to prevent the binding of the non-specific reaction substances to the anti-TAT antibody, and the non-specific reaction substances bound to the IgG-binding peptide bound to the anti-TAT antibody resulting in an increase in the thickness of the layer at the tip of the sensor and a larger change in wavelength shift than when only the non-specific reaction substances bound to the anti-TAT antibody when no non-specific reaction inhibitor was added.

**[Table 4]**

| | Not added | IgG-binding peptide 25 µg/mL | IgG-binding peptide 50 µg/mL | PEG-modified IgG-binding peptide 25 µg/mL | PEG-modified IgG-binding peptide 50 µg/mL |
|---|---|---|---|---|---|
| Response [nm] | 5.7733 | 6.5522 | 6.6331 | 3.0487 | 2.9829 |

### <<Example 4: Comparison of the effects of PEG-modified peptide and antibody fragment>>

The results of Examples 2 and 3 demonstrate that PEG-modified peptides exhibit a non-specific reaction inhibitory effect even though their molecular weight is smaller than that of antibody fragments. This is a new discovery that contradicts the report in Japanese Patent No. 5189098 that antibody fragments have a small molecular weight and therefore a small effect in non-specific reaction inhibition, and that the effect can be achieved by modifying the fragment with PEG to make it larger. Therefore, we compared the effect of inhibiting non-specific reactions when a peptide that binds to non-specific reaction substances was modified with PEG and when an antibody fragment that binds to non-specific reaction substances was modified with PEG.

### [Methods]

A PEG-modified antibody fragment was prepared by chemically modifying Fab' reactive with human IgG with 20 kDa PEG containing a maleimide group at the terminus. Goat anti-human IgG polyclonal IgG (purified from antiserum manufactured by International Immunology Corporation) was digested with pepsin to prepare F(ab'), which was then reduced with 0.1 mol/L TCEP-HCL at 37°C for 210 minutes to prepare Fab'. To the 5 mg/mL Fab' solution, 20 kDa PEG (manufactured by NOF Corporation) with a maleimide group was added, and reacted at 37°C for 30 minutes. The reaction liquid was concentrated to about 5 mg/mL by ultrafiltration, and the buffer was replaced with 0.2 mol/L TBS to obtain PEG-modified anti-human IgG Fab'.

Using the same assay conditions as those described in Example 3, the non-specific reaction inhibitory effects of the PEG-modified peptides described in Examples 2 and 3 and the prepared PEG-modified antibody fragment were compared. The amounts of non-specific reaction inhibitor added were 25 µg/mL, 50 µg/mL, 100 µg/mL, 200 µg/mL, or 400 µg/mL, and the test sample was the same as sample A in Example 3.

### [Results]

The results are shown in Tables 5 and 6. When added in equal amounts, the PEG-modified peptide had a greater effect on inhibiting non-specific reactions than the PEG-modified antibody fragment. This indicates that even a small amount of PEG-modified peptide has a sufficient non-specific reaction inhibitory effect.

**[Table 5]**

| | Amount added | PEG-modified IgG-binding peptide | PEG-modified Anti-human IgG Fab' |
|---|---|---|---|
| Response [nm] | 0 µg/mL | 5.7733 | 5.7733 |
| | 25 µg/mL | 3.0487 | 5.5768 |
| | 50 µg/mL | 2.9829 | 5.5603 |
| | 100 µg/mL | 3.1926 | 5.2206 |

**[Table 6]**

| | Amount added | PEG-modified IgG-binding peptide | PEG-modified Anti-human IgG Fab' |
|---|---|---|---|
| Response [nm] | 0 µg/mL | 6.0744 | 6.0744 |
| | 200 µg/mL | 2.8092 | 5.0809 |
| | 400 µg/mL | 3.0917 | 4.0386 |

### «Example 5: Confirmation of the effect of inhibiting non-specific reaction when the molecular weight of PEG to use modification is increased»

In Japanese Patent No. 5189098, it was confirmed that the larger the molecular weight of the PEG that modified the antibody fragment, the stronger the effect of inhibiting non-specific reactions. Therefore, we investigated the effect of PEG-modified peptides on inhibiting nonspecific reactions when the molecular weight of PEG to use modification was changed.

### [Methods]

PEG-modified IgG-binding peptides were prepared by the method described in Example 1, chemically modified with PEG of 5 kDa, 10 kDa, 20 kDa, or 30 kDa containing a succinimide group at the end.

### [Assay conditions for evaluating the effect of non-specific reaction inhibitors]

The assay was carried out under the same conditions as described in Example 3. As non-specific reaction inhibitors, sample solutions were prepared to which PEG-modified peptides chemically modified with 5 kDa, 10 kDa, 20 kDa, and 30 kDa PEG were added, and the non-specific reaction inhibitory effect of each was confirmed. The amounts of non-specific reaction inhibitors added were 5 µg/mL, 10 µg/mL, or 20 µg/mL, and the test sample was the same as Sample A in Examples 3 and 4.

### [Results]

The results are shown in Table 7. Regardless of the molecular weight of the PEG used, the effect of inhibiting non-specific reactions was observed. It was also confirmed that the effect increased with the molecular weight of the PEG.

**[Table 7]**

| | Amount added | PEG-modified IgG-binding peptide | | | |
|---|---|---|---|---|---|
| | | 5 kDa | 10 kDa | 20 kDa | 30 kDa |
| Response [nm] | 0 µg/mL | 6.0114 | 6.0114 | 6.0114 | 6.0114 |
| | 5 µg/mL | 5.7363 | 5.1166 | 5.5469 | 4.3119 |
| | 10 µg/mL | 5.2651 | 4.4064 | 5.0054 | 3.1534 |
| | 20 µg/mL | 4.5739 | 3.5845 | 3.8339 | 3.1951 |

### «Example 6: Confirmation of non-specific inhibitory effect when using an automatic analyzer»

The effect of inhibiting non-specific reactions was confirmed when measuring with the latex agglutination method using an automatic analyzer.

### [Methods]

The non-specific inhibitory effect of the IgG-binding peptide and PEG-modified IgG-binding peptide used in Example 2 was confirmed by the latex agglutination method.

The measurements were performed using automated operation of an automatic analyzer, STACIA (manufactured by LSI Medience Corporation). In the STACIA measurement, two main operations were carried out. In the first operation, the sample to be measured was diluted with R1 and heated at 37°C for 3.5 minutes. In the second operation, R2 was added to the reaction solution and the mixture was incubated at 37°C for 6 minutes to induce latex agglutination. By optically monitoring the agglutination reaction, the amount of TAT or non-specific reaction substances in the samples to be measured was quantified. In this Example, the non-specific reaction inhibitor was added to R1 so that the concentration after sample addition was 0.05 mg/mL or 0.1 mg/mL, and non-specific reaction substances were absorbed during the first operation. The same volume of PBS(-) was added to R1, which did not contain the nonspecific reaction inhibitor, to avoid the effect of dilution due to additives. The mixture ratio of the measurement sample, R1, and R2 was 20µL:90µL:90µL. Latex agglutination was detected at a wavelength of 700 nm. The measured values were calculated by comparing the absorbance with a calibration curve obtained by measuring TAT of known concentrations. The test sample used was the same as sample A used in Examples 3 to 5.

### [Results]

The results are shown in Table 8. The non-specific inhibitory effect of the PEG-modified IgG-binding peptide was also confirmed in the latex agglutination assay. The reason why the measured value increased with the addition of peptides not modified with PEG was that although the IgG-binding peptide bound to non-specific reaction substances, there was no steric hindrance that was strong enough to prevent the binding of the non-specific reaction substances to the anti-TAT antibody, and the non-specific reaction substances bound to the IgG-binding peptide bound to the anti-TAT antibody, which resulted in a change in absorbance, possibly due to the fact that the aggregates became larger or more complex than when only the non-specific reaction substances bound to the anti-TAT antibody without the addition of the non-specific reaction inhibitor.

**[Table 8]**

| | Amount added | Not added | IgG-binding peptide | PEG-modified IgG-binding peptide |
|---|---|---|---|---|
| Sample A [ng/mL] | 0.05 mg/mL | 32.01 | 37.7 | 10.84 |
| | 0.1 mg/mL | 27.17 | 42.85 | 6.25 |

### «Example 7: Confirmation of the effect of PEG modification of IgG-binding nucleic acid aptamer on inhibiting reaction with anti-human IgG antibody and human IgG»

For nucleic acid aptamers, the reaction inhibitory effect with or without PEG modification was confirmed using a model system to confirm the non-specific inhibitory effect in the same manner as in Example 2.

### [PEG-modified IgG-binding nucleic acid aptamer]

The nucleic acid aptamer used was a human IgG-binding nucleic acid aptamer of approximately 7.5 kDa (nucleic acid sequence:
N(6)_ggAGGU(F)GcU(F)ccgaaaGGA(L)aC(F)U(F)cc (upper case letters = RNA, lower case letters = DNA, N(F) = 2'-Fluoro RNA, N(L) = LNA, N(6) = Amino C6 linker) that has been reported for the purpose of purifying human IgG. The PEG-modified nucleic acid aptamer was prepared by binding one molecule of PEG to one molecule of nucleic acid aptamer via the amino group at the N-terminus (nucleic acid sequence: P_N(6)_ggAGGU(F)GcU(F)ccgaaaGGA(L)aC(F)U(F)cc (upper case letters = RNA, lower case letters = DNA, N(F) = 2'-Fluoro RNA, N(L) = LNA, N(6) = Amino C6 linker, P = PEG (Jenkem ME 20kDa)).

### [Methods]

The test sample and measurements were performed under the same assay conditions as in Example 2. In this Example, the test sample and a non-specific reaction inhibitor were allowed to coexist in the measurement sample, and human IgG was absorbed in the sample solution. The measurements were carried out according to the operating manual attached to the OctetR2.

Anti-human IgG polyclonal rabbit antibody (manufactured by Dako) was diluted with PBS(-) to 25 µg/mL to prepare an anti-human IgG antibody solution. The sample solution used was human IgG at 1 mg/mL in PBS(-), with 20 µL of this test sample, and either the IgG-binding nucleic acid aptamer or the IgG-binding nucleic acid aptamer modified with 20 kDa PEG added to a final concentration of 0.3 mg/mL, and then prepared with reaction buffer (145 mmol/L NaCl, 5.4 mmol/L KCl, 0.8 mmol/L MgCl₂, 1.8 mmol/L CaCl₂, 20 mmol/L Tris (pH 7.6), 0.05% Tween 20) to make a total volume of 200 µL.

### [Results]

The results are shown in Table 9. The IgG-binding nucleic acid aptamer, when modified with PEG, had a stronger inhibitory effect on the reaction between the anti-human IgG antibody and human IgG than the unmodified case. In addition, the IgG-binding nucleic acid aptamer was approximately 7.5 kDa, and even when modified with 20 kDa PEG, the molecular weight was 27.5 kDa, which was smaller than that of the antibody fragment, but it showed a reaction inhibitory effect.

**[Table 9]**

| | Not added | IgG-binding nucleic acid aptamer 0.3 mg/mL | PEG-modified IgG-binding nucleic acid aptamer 0.3 mg/mL |
|---|---|---|---|
| Human IgG [nm] | 7.9093 | 7.6691 | 4.9568 |

### INDUSTRIAL APPLICABILITY

The non-specific reaction inhibitor of the present invention can be used in immunological measurements.

### [FREE TEXT IN SEQUENCE LISTING]

The amino acid sequences represented by SEQ ID NO: 1 and SEQ ID NO: 2 in the sequence listing are a human IgG-binding peptide and a human IgA-binding peptide, respectively.

[SEQUENCE LISTING]

## Claims

1. A non-specific reaction inhibitor for immunological measurement, comprising a complex of a peptide or nucleic acid molecule that specifically binds to a non-specific reaction substance and a polymer compound.

2. The non-specific reaction inhibitor according to claim 1, wherein the substance that specifically binds to the non-specific reaction substance is a chemically synthesizable substance.

3. The non-specific reaction inhibitor according to claim 1 or 2, wherein the complex is 50 kDa or less.

4. The non-specific reaction inhibitor according to any one of claims 1 to 3, wherein the polymer compound is polyethylene glycol.

5. The non-specific reaction inhibitor according to any one of claims 1 to 4, wherein the non-specific reaction substance is an immunoglobulin.

6. An immunological measurement method, **characterized by** using a complex of a peptide or nucleic acid molecule that specifically binds to a non-specific reaction substance and a polymer compound.

7. The immunological measurement method according to claim 6, wherein the immunological measurement method is a latex agglutination photometric assay, an immunonephelometric assay, an immunoturbidimetric assay, a chemiluminescent immunoassay, an enzyme immunoassay, a fluorescent immunoassay, or a radioimmunoassay.

8. An immunological measurement reagent, comprising a complex of a peptide or nucleic acid molecule that specifically binds to a non-specific reaction substance and a polymer compound.

9. The immunological measurement reagent according to claim 8, wherein the complex of a peptide or nucleic acid molecule that specifically binds to a non-specific reaction substance and a polymer compound is contained in a stabilizing reagent.
